Europäisches Patentamt

European Patent Office

Office européen des brevets

(11) Publication number: 0 413 421 A1

## (12) EUROPEAN PATENT APPLICATION

(21) Application number: 90306648.8

(51) Int. Cl.5: C07K 7/10, A23L 1/236

(22) Date of filing: 19.06.90

(30) Priority: 19.06.89 JP 156310/89

(43) Date of publication of application:
20.02.91 Bulletin 91/08

(84) Designated Contracting States:
DE FR GB NL

(71) Applicant: AJINOMOTO CO., INC.
5-8, Kyobashi 1-chome, Chuo-ku
Tokyo 104(JP)

(72) Inventor: Komura, Masanori c/o Central
Research Lab.
Ajinomoto Co., Inc., 1-1 Suzuki-cho,
Kawasaki-ku
Kawaaki-shi, Kanagawa-ken(JP)
Inventor: Nio, Noriki c/o Central Research
Lab.
Ajinomoto Co., Inc., 1-1 Suzuki-cho,
Kawasaki-ku
Kawaaki-shi, Kanagawa-ken(JP)
Inventor: Ariyoshi, Yasuo c/o Central
Research Lab.
Ajinomoto Co., Inc., 1-1 Suzuki-cho,
Kawasaki-ku
Kawaaki-shi, Kanagawa-ken(JP)

(74) Representative: Stuart, Ian Alexander et al
MEWBURN ELLIS & CO. 2/3 Cursitor Street
London EC4A 1BQ(GB)

(54) Monellin analog and sweetening composition containing it.

(57) The invention concerns a protein which has an A chain and/or a B chain and salts and derivatives of the protein. The chains comprise part or all of the structural formula:

A chain: Arg-Glu-Ile-Lys-Gly-Tyr-Glu-Tyr-Gln-Leu-Tyr-Val-Tyr-Ala-Ser-Asp-Lys-Leu-Phe-Arg-Ala-Asp-Ile-Ser-Glu-Asp-Tyr-Lys-Thr-Arg-Gly-Arg-Lys-Leu-Leu-Arg-Phe-Asn-Gly-Pro-Val-Pro-Pro-Pro

B chain: Gly-Glu-Trp-Glu-Ile-Ile-Asp-Ile-Gly-Pro-Phe-Thr-Gln-Asn-Leu-Gly-Lys-Phe-Ala-Val-Asp-Glu-Glu-Asn-Lys-Ile-Gly-Gln-Tyr-Gly Arg-Leu-Thr-Phe-Asn-Lys-Val-Ile-Arg-Pro-Cys-Met-Lys-Lys-Thr-Ile-Tyr-Glu-Asn-Glu.

The protein has a sweet taste of approximately 4000 times (weight basis) that of sucrose. Therefore the protein has use as a sweetener.

Sweetener formulations comprising the protein are also provided, as are processes for making the protein.

Fig.1.

B Chain

| | | 1 10 |
|---|---|---|
| Invention | | Gly-Glu-Trp-Glu-Ile-Ile-Asp-Ile-Gly-Pro-Phe-Thr-Gln-Asn-Leu-Gly-Lys-Phe-Ala- |
| Natural | 57% | Gly-Glu-Trp-Glu-Ile-Ile-Asp-Ile-Gly-Pro-Phe-Thr-Gln-Asn-Leu-Gly-Lys-Phe-Ala- |
| Mixture | 24% | Glu-Trp-Glu-Ile-Ile-Asp-Ile-Gly-Pro-Phe-Thr-Gln-Asn-Leu-Gly-Lys-Phe-Ala- |
| | 19% | Thr-Gly-Glu-Trp-Glu-Ile-Ile-Asp-Ile-Gly-Pro-Phe-Thr-Gln-Asn-Leu-Gly-Lys-Phe-Ala- |

| | | 20 30 |
|---|---|---|
| Invention | | Val-Asp-Glu-Glu-Asn-Lys-Ile-Gly-Gln-Tyr-Gly-Arg-Leu-Thr-Phe-Asn-Lys-Val-Ile-Arg |
| Natural | 57% | Val-Asp-Glu-Glu-Asn-Lys-Ile-Gly-Gln-Tyr-Gly-Arg-Leu-Thr-Phe-Asn-Lys-Val-Ile-Arg |
| Mixture | 24% | Val-Asp-Glu-Glu-Asn-Lys-Ile-Gly-Gln-Tyr-Gly-Arg-Leu-Thr-Phe-Asn-Lys-Val-Ile-Arg |
| | 19% | Val-Asp-Glu-Glu-Asn-Lys-Ile-Gly-Gln-Tyr-Gly-Arg-Leu-Thr-Phe-Asn-Lys-Val-Ile-Arg |

| | | 40 50 |
|---|---|---|
| Invention | | Pro-Cys-Met-Lys-Lys-Thr-Ile-Tyr-Glu-Asn-Glu |
| Natural | 57% | Pro-Cys-Met-Lys-Lys-Thr-Ile-Tyr-Glu-Glu-Asn |
| Mixture | 24% | Pro-Cys-Met-Lys-Lys-Thr-Ile-Tyr-Glu-Glu-Asn |
| | 19% | Pro-Cys-Met-Lys-Lys-Thr-Ile-Tyr-Glu-Glu-Asn |

## SWEETENERS

The present invention relates to sweeteners. In particular, the present invention relates to a novel protein and a sweetener formulation comprising said novel protein.

A protein, monellin, which is obtained from the fruits of Dioscorephyllum cumminsii (Stapf) Diels has a strong sweetness of approximately 3000 times (weight basis) greater than that of sucrose (Morris, J.A. & Cagan, R.H. (1972), Biochim. Biophys. Acta, 261, 114-122 and Van der Wel, H. (1972), FEBS Lett., 21, 88-90). Monellin is composed of an A chain having 44 amino acid residues and a B chain having 50 amino acid residues. To date, two kinds of the primary structure have been proposed for both the A chain and the B chain (Bohak, Z. & Li, S.L. (1976) Biochim. Biophys. Acta, 427, 153-170, Frank, G. & Zuber, H. (1976) Hoppe-Seyler's Z. Physiol. Chem., 357, 585-592 and Hudson, G. & Biemann, K. (1976), Biochem. Biophys. Res. Commun., 71, 212-220). The present applicants have now confirmed and identified the structure of natural monellin. When HPLC analysis is carried out on natural monellin, two peaks are shown for the A chain and one peak is shown for the B chain. Therefore the B chain was thought to comprise one polypeptide. However, by measuring ESI (electro-spray ionisation) MS, the present applicants have found that the B chain has three amino acid sequences. In natural monellin the applicants have found that the A chain comprises a mixture of:

|  | 1 | 44 |
|---|---|---|
| 90% | Arg-Glu- ..... -Pro-Pro; and |  |
| 10% | Phe-Arg-Glu- ..... -Pro-Pro. |  |

Similarly, in natural monellin the B chain has been found to comprise a mixture of:

| 57% | Gly-Glu- ..... -Glu-Glu-Asn; |
|---|---|
| 24% | Glu- ..... -Glu-Glu-Asn; and |
| 19% | Thr-Gly-Glu- ..... -Glu-Glu-Asn. |

The applicants believe that in natural monellin, one of the two possible A chains and one of the three possible B chains associate to form a complex.

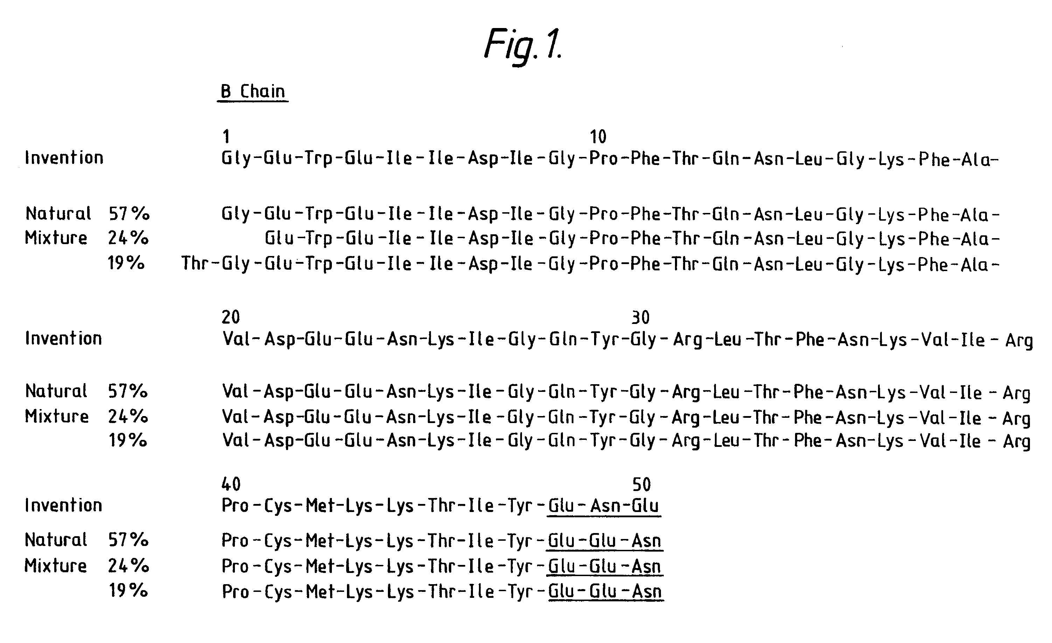

The sequence of the B chain of natural monellin as compared to the protein of the present invention is shown in Figure 1. The B chain of the protein of the present invention differs from the 57% B chain of natural monellin, in amino acids 48, 49 and 50 as shown in the figure. The synthetic protein of the present invention is sweeter than natural monellin.

Although, the protein has been synthesized generally by genetic engineering techniques, there have been no reports of synthesis of a pure monellin protein by chemical solid phase synthesis.

The present invention aims at:

1) synthesizing the protein monellin in a simple manner by chemical solid phase synthesis;
2) determining the true primary structural formula of monellin; and
3) further establishing the chemical synthesis of the protein by the solid phase method.

The present applicants have entered into extensive investigations to achieve the above. The applicants have determined the true structure of monellin by using the techniques of mass spectroscopy, protein sequencing and HPLC. Broadly, the applicants have identified the true structure of natural monellin by synthesizing monellin analogues and then comparing them to natural monellin by peptide mapping, HPLC and mass spectrum techniques. In so doing, the applicants have found a novel synthetic protein which surprisingly, is much sweeter than natural monellin.

The applicants were successful in their approach by:

1) protecting the $\alpha$-amino group with a Fmoc group (see later for full definitions of these abbreviations) which can be removed with a weak base; and

2) using a protective group for the side chain which can be removed by treatment with an acid.

More specifically: a But group is used for protecting hydroxy carboxyl groups; a Mtr group is used for protecting the guanidino group of arginine; a Boc group is used for protecting the ö-amino group of lysine; and an Mbh group is used for protecting the sulfhydryl group of cysteine. Furthermore, methionine is introduced as methionine oxide and the methionine oxide is reduced at the final stage of removing the protective group to revert to a methionine residue. The A and B chains shown by the structural formulae below can be synthesized by the chemical solid phase synthesis methods, and the applicants have found that the protein comprising these A and B chains exhibits a sweet taste. The present invention has thus been accomplished.

In the present invention the primary amino acid structure of the A and B chains is as shown below.

A chain: Arg-Glu-Ile-Lys-Gly-Tyr-Glu-Tyr-Gln-Leu-Tyr-Val-Tyr-Ala-Ser-Asp-Lys-Leu-Phe-Arg-Ala-Asp-Ile-Ser-Glu-Asp-Tyr-Lys-Thr-Arg-Gly-Arg-Lys-Leu-Leu-Arg-Phe-Asn-Gly-Pro-Val-Pro-Pro-Pro-

B chain: Gly-Glu-Trp-Glu-Ile-Ile-Asp-Ile-Gly-Pro-Phe-Thr-Gln-Asn-Leu-Gly-Lys-Phe-Ala-Val-Asp-Glu-Glu-Asn-Lys-Ile-Gly-Gln-Tyr-Gly-Arg-Leu-Thr-Phe-Asn-Lys-Val-Ile-Arg-Pro-Cys-Met-Lys-Lys-Thr-Ile-Tyr-Glu-Asn-Glu.

The present invention provides a protein which comprises part or all of an A chain and/or a B chain as shown above, and salts and derivatives of said protein. The present invention also provides part or all of the A and/or B chains as shown above synthesized by chemical solid phase synthesis for said protein.

According to the solid phase synthesis method of the present invention, condensation is carried out in the presence of a condensing agent such as DCC. Alternatively, a symmetric acid anhydride synthesized from a protected amino acid and DCC can be used. After completion of the condensation, the protective group is removed and the bond between the C terminus of the protein and the resin is cleaved. The A chain and B chain of the present invention can be purified according to conventional techniques. For example, by ion exchange chromatography, reverse phase chromatography, affinity chromatography, etc.

The thus synthesized A chain and B chain as described above, are then mixed in equimolar amounts in an aqueous solution to form the complex. The complex is separated and purified by chromatography as described above. The thus obtained protein is crystallized from, for example a water-polyethylene glycol mixture. The thus obtained protein shows a sweetness of about 4000 times that of sucrose in a weight ratio. Individually, the A chain and B chains do not show any sweetness.

The protein of the present invention can be used in a fashion similar to the use of ordinary low calorie sweeteners already available in the art.

Examples

Hereafter the present invention is described in more detail by way of example only and not by way of limitation with reference to the examples.

Abbreviations used in the specification mean the following:

Ala alanine (hereafter all amino acids are in the L-form)
Arg arginine
Asn asparagine
Asp aspartic acid
Cys cysteine
Gln glutamine
Glu glutamic acid
Gly glycine
Ile isoleucine
Leu leucine
Lys lysine
Met methionine
Phe phenylalanine
Pro proline
Ser serine
Thr threonine
Tyr tyrosine
Val valine
Fmoc fluoren-9-ylmethoxycarbonyl
But tertiary butyl

OBut tertiary butyl ester
Mtr 4-methoxy-2,3,6-trimethylbenzenesulfonyl
Boc tertiary butoxycarbonyl
Mbh 4,4´-dimethoxybenzhydryl
MBzl p-methoxybenzyl
DCC dicyclohexylcarbodiimide
TFA trifluoroacetic acid
HOBt 1-hydroxybenzotriazole
TMSBr trimethylsilyl bromide
DMF N,N-dimethylformamide
NMP N-methylpyrrolidone
HPLC high performance liquid chromatography
TLC thin layer chromatography

## Example 1 - Synthesis of the A Chain

200 mg of Fmoc-Pro-resin (p-methoxybenzyl alcohol resin in which Fmoc-Pro has been introduced in a ratio of 0.53 mmols/g) was charged in a reactor of 50 ml volume. DMF (15 ml) was added to the mixture to create a suspension. The suspension was shaken for 15 minutes to swell the Fmoc-Pro-resin.

Peptide synthesis was carried out by the DCC/HOBt method using 3 molar equivalents (0.318 mmol) of protected amino acid, 3.3 molar equivalents (0.350 mmol) of DCC and 3.6 molar equivalents (0.382 mmol) of HOBt or by using symmetric acid anhydride prepared by treating 6 molar equivalents (0.636 mmol) of protected amino acid and 3 molar equivalents (0.381 mmol) of DCC in methylene chloride at $0^\circ$C for 20 minutes. Each condensation was performed until the Kaiser test (Kaiser, E., Colescott, R.L., Bossinger, C.D. & Cook, P.I.(1970), Anal. Biochem., 34, 595-598) became negative. When the test did not become negative even though the condensation was repeated, "capping", in which elongation of the unreacted peptide was discontinued by treating with acetic anhydride and pyridine was carried out.

The following synthesis cycle was used.

(a) Removal of Fmoc: (i) shaking in DMF (15 ml) for a! minute to swell the resin. (ii) treating twice (3 minutes + 10 minutes) with 50% piperidine in DMF (15 ml) while shaking.

(b) Washing: (i) 4 times with DMF (15 ml). (ii) twice with isopropanol (15 ml). (iii) confirming that the Kaiser test is completely positive. If the removal of Fmoc was incomplete, the procedures (a) and (b) were repeated.

(c) Condensation: (i) shaking in DMF (15 ml) for a minute to swell the resin. The procedure was repeated twice. (ii) Fmoc-amino acid derivative (0.318 mmol) and HOBt (0.382 mmol) were dissolved in a solvent mixture of methylene chloride (9 ml) and DMF (1 ml), and 1 M DCC solution (0.35 ml, in methylene chloride) was added to the solution. (iii) shaking for 70 minutes.

(d) Washing: (i) once with DMF (15 ml). (ii) twice with isopropanol (15 ml). (iii) the condensation was performed until the Kaiser test became negative. If it was positive, the procedure (c) was repeated. When it was positive, even though the reaction had been repeated, the symmetric acid anhydride method was applied or capping was conducted.

In the cycle described above, Fmoc was removed from the Fmoc-Pro-resin and Fmoc-Pro was introduced. When it was attempted to remove Fmoc group from the thus obtained Fmoc-Pro-Pro-resin with piperidine, it was noted that the dipeptide was readily cleaved. Accordingly, synthesis of this portion was performed by introducing Fmoc-Pro-Pro (0.318 mmol) after removing the protective group of the Fmoc-pro-resin and the resulting Fmoc-Pro-Pro-resin was provided in the subsequent step of removing the protective group.

After completion of the final condensation step, removal of protective group and purification were performed by the following treatments.

After the peptide-resin was washed 3 times with methylene chloride (15 ml), the resin was stirred in methylene chloride-anisole-thiophenol-TFA (12.3 : 2.0 : 0.7 : 20; v/v, 35 ml) for 2 hours at room temperature. After filtering, the filtrate was concentrated at a hot bath temperature of $50^\circ$C under reduced pressure to a small volume. Ether was added to give precipitates. The precipitates were taken out by filtration and dried giving a yield of 518 mg. The thus obtained peptide was further dissolved in thiophenol-thioanisole-TFA (0.5 : 1.5 : 13.5; v/v, 15.5 ml). The solution was stirred at room temperature for 5 hours. After the reaction, a sufficient volume of ether was added to the solution and the formed precipitates were taken by filtration and dried. The yield was 485 mg. The thus obtained crude peptide was applied to HPLC (Inertsil, ODS, using a column of Gas Chromatography Industry Co., Ltd.). The desired fraction of the A chain was fractionated, concentrated to a small volume and then lyophilized. The yield was 78.6 mg.

Measurement of the molecular weight with a FAB mass analyzer gave m/z: 5248.3 $(M + H)^+$, which was coincident with calculated value m/z: 5248.8 $(M + H)^+$. Furthermore, the product was hydrolyzed in 6N-HCl aqueous solution for amino acid analysis, which gave a ration of: Asp, 4.00 (4); Thr, 1.01 (1); Ser, 0.81 (2); Glu, 3.96 (4); Pro, 4.03 (4); Gly, 3.02 (3); Ala, 2.03 (2); Val, 1.97 (2); Ile, 1.90 (2); Leu, 4.01 (4); Tyr, 4.84 (5); Phe, 2.01 (2); Lys, 3.95 (4); Arg, 4.99 (5); which was also coincident with the calculated data.

The figures within parentheses indicate calculated data. It was thus confirmed that the desired peptide was synthesized.

With regard to its purity, it was also confirmed by TLC and HPLC that the peptide was synthesized in good purity.

Example 2 - Synthesis of B Chain

1376 mg of Fmoc-Glu(OBut)-resin (p-methoxybenzyl alcohol resin in which Fmoc-Glu(OBut) was introduced in a ratio of 0.41 mmols/g) was charged in a reactor of 50 ml volume. DMF (15 ml) was added to the mixture to create a suspension. The suspension was shaken for 15 minutes to swell the resin. The synthesis cycle was basically the same as the synthesis process described for the A chain, but slightly modified by reference to the finding obtained by preliminary synthesis of B chain.

(a) Removal of Fmoc: (i) Fmoc of Asn(Mbh) at the 49-position was removed with 20% piperidine in DMF. (ii) reaction time: 3 minutes + 10 minutes for 50 to 40 residues; 3 minutes + 15 minutes for 39 to 15 residues; 3 minutes + 10 minutes for 14 to 1 residues.

(b) Condensation: (i) condensation of 50-40, 36-34 and 32 residues was performed by the DCC/HOBt method. That is, Fmoc-amino acid derivative (1.128 mmol) and HOBt (1.354 mmol) were dissolved in a solvent mixture of methylene chloride (9 ml) and DMF (1 ml) and 1 M DCC (1.24 ml, in methylene chloride) was added to the solution followed by shaking at room temperature for 90 minutes. Condensation of 39-37, 33, 31, 12-10 and 8-2 residues was carried out by the symmetric acid anhydride method. The symmetric acid anhydride was prepared by dissolving Fmoc-amino acid derivative (2.256 mmols) in a solvent mixture of DMF (5 ml) and methylene chloride (5 ml), adding 1 M DCC (1.23 ml, in methylene chloride) to the solution and treating at 0°C for 20 minutes. With respect to the 39, 33 and 31 residues, the reaction was conducted for 4 hours. The 39, 38, 33, 31 and 6 residues showed positive in the Kaiser test, so that the second condensation was necessary. The second condensation was carried out by the DCC/HOBt method. That is, Fmoc-amino acid derivative (1.128 mmol) and HOBt (1.354 mmol) were dissolved in NMP (10 ml) and 1 M DCC (1.24 ml, in methylene chloride) was added to the solution followed by shaking for 2 hours. The mixture was then allowed to stand overnight. Condensation of the 30-13 and 9 residues was carried out by the DCC/HOBt method. That is, Fmoc-amino acid derivative (1.692 mmol) and HOBt (2.030 mmol) were dissolved in NMP (10 ml) and 1 M DCC (1.86 ml, in methylene chloride) was added to the solution followed by shaking for 3 hours. The second condensation was necessary for the 28 and 13 residues. That is, Fmoc-amino acid derivative (1.128 mmol) and HOBt (1.354 mmol) were dissolved in NMP (10 ml) and 1 M DCC (1.24 ml, in methylene chloride) was added to the solution followed by shaking for 2 hours. The mixture was then allowed to stand overnight. Lastly, Boc-Gly (1.692 mmol) was treated with HOBt (2.030 mmol) and 1 M DCC (1.86 ml, in methylene chloride) in NMP (10 ml) followed by shaking for 3 hours.

Following the last condensation step, removal of the protective group and purification were carried out by the following treatments.

The peptide-resin was washed twice with DMF (15 ml) and 4 times with isopropanol (15 ml) and then dried. The yield was 3510 mg. From the peptide-resin, 1510 mg was taken. After further washing 3 times with methylene chloride (15 ml), methylene chloride-anisole-m-cresol-1,2-ethanedithiol-TFA (12.3 : 2.0 : 0.5 : 0.2 : 20; v/v, 35 ml) was added followed by shaking for 2 hours at room temperature. After filtering, the filtrate was concentrated under reduced pressure to a small volume. Ether was added to the concentrate. The precipitates were taken out by filtration and dried. The yield was 807 mg. The crude peptide was dissolved in thioanisole- m-cresol-1,2-ethanedithiol-TFA (2.35 : 0.5 : 0.2 : 14.31; v/v, 17.36 ml). The solution was stirred at room temperature for 10 minutes and then cooled with ice. Whilst stirring, TMSBr (2.64 ml) was drop-wise added to the solution. Stirring was continued for 4 hours under ice cooling. A sufficient volume of ether was added to the reaction solution and the formed precipitates were taken by filtration and dried. The precipitates were dissolved in water (40 ml). Whilst stirring under ice cooling, the pH of the solution was adjusted to 8 with triethylamine. B-mercaptoethanol (400 μl) and 1 M ammonium fluoride (800

μl) were added to the solution. Whilst stirring under ice cooling, acetic acid was added to the mixture to adjust the pH to 5. The formed precipitates were filtered and suspended in 20% acetonitrile (20 ml) containing 0.05% TFA followed by centrifugation at 10,000 rpm for 30 minutes. The supernatant was subjected to HPLC as described for the A chain. The desired fraction of the B chain was fractionated and concentrated under reduced pressure to a small volume and then lyophilized. The yield was 1.84 mg. The product was further subjected to the same HPLC for purification. Thus, pure B chain was obtained. The yield was 14.9 mg. Measurement of the molecular weight with a FAB mass analyzer gave m/z: 5831.7 (M + H)$^+$, which was coincident with the calculated value m/z: 5832.0 (M + H)$^+$. Furthermore, the product was hydrolyzed in 6N-HCl aqueous solution for amino acid analysis, which gave a ratio of: Asp, 6.16 (6); Thr, 2.87 (3); Glu, 7.71 (8); Pro, 2.05 (2); Gly, 5.19 (5); Ala, 1.05 (1); Val, 1.78 (2); Met, 0.93 (1); Ile, 5.51 (6); Leu, 2.09 (2); Tyr, 2.05 (2); Phe, 3.14 (3); Lys, 5.04 (5); Arg, 2.09 (2); which was also coincident with the calculated data. The figures within parentheses indicate calculated data.

It was thus confirmed that the desired peptide was synthesized. Trp and Cys were not determined since they were decomposed.


Example 3 - Synthesis of Protein

A chain (2.53 mg) synthesized above was dissolved in 0.1% acetic acid (631 μl) and 607 μl was taken from the solution. B chain (2.43 mg) was dissolved in the solution (607 μl) containing this A chain. The resulting solution was equilibrated at room temperature for 20 hours. The mixture was subjected to HPLC (TSK gel Phenyl 5PW, using a column of Toso Co., Ltd.) to fractionate the desired protein fraction. The fraction was then lyophilized. This protein was dissolved in water (400 μl). After shaking at room temperature for 2 hours, the solution was subjected to HPLC as described above, to fractionate the protein fraction. The fraction was lyophilized. The yield was 2.13 mg. This protein showed a single peak in HPLC.

Crystallization was performed by, for example, equilibrating a solution containing 4 mg/ml of protein, 14% (w/w) polyethylene glycol and 10 mM phosphate buffer with 28% (w/w) polyethylene glycol at 4°C. Plate-like crystals were obtained. This fact also confirmed that pure protein had been synthesized.


Example 4 - Sweet Taste Test

The protein obtained as above was dissolved in water. Comparison with 0.6% sucrose aqueous solution by a panel of 5 untrained people showed that the protein solution had a sweet taste of about 4000 times (weight ratio) that of sucrose.

From the foregoing results, the protein of the present invention can be seen to be useful as a sweetener and the protein will have use in the food and medical industries.


**Claims**

1. A protein comprising an A chain and/or a B chain, and salts and derivatives of said protein, and wherein which chains comprise part or all of the structural formulae shown below:

A chain: Arg-Glu-Ile-Lys-Gly-Tyr-Glu-Tyr-Gln-Leu-Tyr-Val-Tyr-Ala-Ser-Asp-Lys-Leu-Phe-Arg-Ala-Asp-Ile-Ser-Glu-Asp-Tyr-Lys-Thr-Arg-Gly-Arg-Lys-Leu-Leu-Arg-Phe-Asn-Gly-Pro-Val-Pro-Pro-Pro

B chain: Gly-Glu-Trp-Glu-Ile-Ile-Asp-Ile-Gly-Pro-Phe-Thr-Gln-Asn-Leu-Gly-Lys-Phe-Ala-Val-Asp-Glu-Glu-Asn-Lys-Ile-Gly-Gln-Tyr-Gly Arg-Leu-Thr-Phe-Asn-Lys-Val-Ile-Arg-Pro-Cys-Met-Lys-Lys-Thr-Ile-Tyr-Glu-Asn-Glu.

2. A protein according to claim 1 which comprises A and B chains in a ratio of 1:1.

3. A protein according to claim 1 or claim 2 wherein said A and/or B chains are synthesized by chemical solid phase synthesis.

4. A protein according to any one of the preceding claims or a salt or derivative of said protein which has a sweet taste of approximately 4000 times (weight basis) that of sucrose.

5. A sweetener formulation which comprises a protein according to any one of the preceding claims and/or a salt or derivative of said protein.

6. A chemical solid phase synthesis process for making a protein according to any one of the preceding claims which comprises:

a) protecting the α-amino groups with Fmoc groups which can be removed with a weak base; and

b) using protective group for side chains which can be removed by treatment with an acid.

7. A process according to claim 6 wherein:

i) a But group is used for protecting hydroxy and carboxyl groups;

ii) a Mtr group is used for protecting the guanidino group of arginine;

iii) a Boc group is used for protecting the ö-amino group of lysine; and/or

iv) a Mbh group is used for protecting the sulfhydryl group of cysteine.

8. A process according to claim 6 or claim 7 wherein methionine is introduced as methionine oxide and the methionine oxide is reduced at the stage of removal of the protective group to revert to a methionine residue.

# Fig.1.

EP 0 413 421 A1

**B Chain**

|           |      | 1                                                                                             10 |
|-----------|------|---------------------------------------------------------------------------------------------------|
| Invention |      | Gly-Glu-Trp-Glu-Ile-Ile-Asp-Ile-Gly-Pro-Phe-Thr-Gln-Asn-Leu-Gly-Lys-Phe-Ala- |

Natural 57%    Gly-Glu-Trp-Glu-Ile-Ile-Asp-Ile-Gly-Pro-Phe-Thr-Gln-Asn-Leu-Gly-Lys-Phe-Ala-
Mixture 24%         Glu-Trp-Glu-Ile-Ile-Asp-Ile-Gly-Pro-Phe-Thr-Gln-Asn-Leu-Gly-Lys-Phe-Ala-
         19%   Thr-Gly-Glu-Trp-Glu-Ile-Ile-Asp-Ile-Gly-Pro-Phe-Thr-Gln-Asn-Leu-Gly-Lys-Phe-Ala-

            20                                30

Invention    Val-Asp-Glu-Glu-Asn-Lys-Ile-Gly-Gln-Tyr-Gly-Arg-Leu-Thr-Phe-Asn-Lys-Val-Ile-Arg

Natural 57%    Val-Asp-Glu-Glu-Asn-Lys-Ile-Gly-Gln-Tyr-Gly-Arg-Leu-Thr-Phe-Asn-Lys-Val-Ile-Arg
Mixture 24%    Val-Asp-Glu-Glu-Asn-Lys-Ile-Gly-Gln-Tyr-Gly-Arg-Leu-Thr-Phe-Asn-Lys-Val-Ile-Arg
         19%    Val-Asp-Glu-Glu-Asn-Lys-Ile-Gly-Gln-Tyr-Gly-Arg-Leu-Thr-Phe-Asn-Lys-Val-Ile-Arg

            40                                50

Invention    Pro-Cys-Met-Lys-Lys-Thr-Ile-Tyr-<u>Glu-Asn-Glu</u>

Natural 57%    Pro-Cys-Met-Lys-Lys-Thr-Ile-Tyr-<u>Glu-Glu-Asn</u>
Mixture 24%    Pro-Cys-Met-Lys-Lys-Thr-Ile-Tyr-<u>Glu-Glu-Asn</u>
         19%    Pro-Cys-Met-Lys-Lys-Thr-Ile-Tyr-<u>Glu-Glu-Asn</u>

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 810 265 (THE REGENTS OF THE UNIVERSITY OF CALIFORNIA) * Page 2, lines 1-28; figure 1 * | 1-8 | C 07 K 7/10 A 23 L 1/236 |

TECHNICAL FIELDS SEARCHED (Int. Cl.5)

C 07 K
A 23 L

The present search report has been drawn up for all claims

| Place of search | Date of completion of the search | Examiner |
|---|---|---|
| THE HAGUE | 14-09-1990 | GROENENDIJK M.S.M. |

EPO FORM 1503 03.82 (P0401)